(19) Europäisches Patentamt
European Patent Office
Office européen des brevets

(11) **EP 3 613 339 A1**

(12) **EUROPEAN PATENT APPLICATION**

(43) Date of publication:
**26.02.2020 Bulletin 2020/09**

(51) Int Cl.:
***A61B 5/02*** *(2006.01)*      ***A61B 5/021*** *(2006.01)*
***A61B 5/0215*** *(2006.01)*

(21) Application number: **18189998.0**

(22) Date of filing: **21.08.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
- **VAN DER HORST, Arjen
5656 AE Eindhoven (NL)**
- **KUENEN, Maarten Petrus Joseph
5656 AE Eindhoven (NL)**
- **MUELLER, Manfred
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **RENAL DENERVATION PREPARATION**

(57) The present invention relates to the context of renal denervation. In order to provide further improvement in relation to the outcome of renal denervation, an apparatus (10) for renal denervation preparation is provided that comprises an input unit (12), a processing unit (14) and an output unit (16). The input unit is configured to receive first artery parameter data and second artery parameter data. The first artery parameter data relates to artery stiffness of a renal artery part of a subject, and the second artery parameter data relates to artery stiffness of a non-renal artery part of the subject. The processing unit is configured to calculate a respective vessel stiffness for the renal artery part based on the first artery parameter data, and to calculate a respective vessel stiffness for the non-renal artery part based on the second artery parameter data, and also to determine a response parameter for renal denervation based on the vessel stiffness for the renal artery part and the vessel stiffness for the non-renal artery part. The output unit is then configured to provide the response parameter.

Fig. 4

**Description**

FIELD OF THE INVENTION

The present invention relates to the context of renal denervation, and relates in particular to a device for renal denervation preparation, to a system for renal denervation assessment and to a method for renal denervation preparation.

BACKGROUND OF THE INVENTION

Hypertension is related to health risks. As an example, one of the causes of hypertension is sympathetic overdrive affecting renal arterial tone. Renal denervation is one option to block the respective neural signals and therefore lower the blood pressure. However, the efficacy of renal denervation can vary between patients. For example, WO 2017 198490 A1 relates to determining the velocity of the pressure/flow pulse, i.e. pulse wave velocity inside the main renal artery, for further improving the stratification of patients in view of the outcome of renal denervation. However, it has been shown that patients respond differently to renal denervation (RDN). Besides the stiffening of the renal arteries due to electrical neural signals, also other factors can influence arterial stiffness.

SUMMARY OF THE INVENTION

There may thus be a need to provide further improvement in relation to the outcome of renal denervation.

The object of the present invention is solved by the subject-matter of the independent claims; further embodiments are incorporated in the dependent claims. It should be noted that the following described aspects of the invention apply also for the device for renal denervation preparation, for the system for renal denervation assessment and for the method for renal denervation preparation.

According to the present invention, a method for renal denervation preparation, comprising the following steps:

    a) receiving first artery parameter data and second artery parameter data;
    wherein the first artery parameter data relates to artery stiffness of a renal artery part; and
    wherein the second artery parameter data relates to artery stiffness of a non-renal artery part;
    b) calculating a respective vessel stiffness for the renal artery part based on the first artery parameter data; and calculating a respective vessel stiffness for the non-renal artery part based on the second artery parameter data;
    c) determining a response parameter for renal denervation based on the vessel stiffness for the renal artery part and the vessel stiffness for the non-renal artery part; and
    d) providing the response parameter.

This parameter provides further information to medical staff for being able to distinguish arterial stiffening due to sympathetic overdrive from systemic arteriosclerosis. Hence, the prediction and assessment of possible renal denervation may be improved.

According to an example, the first artery parameter data and the second artery parameter data are based on pulse wave velocity (PWV). Alternatively, or in addition, the first artery parameter data and the second artery parameter data are based on measurements from the same point in time.

According to an example, for step a), the first artery parameter data is measured at a first location in a vessel structure of an object and the second parameter data is measured at a second location in a vessel structure of an object. The first data is measured in a renal artery part and the second data is measured in a non-renal artery part.

According to the present invention, also a device for renal denervation preparation is provided. The device comprises an input unit, a processing unit and an output unit. The input unit is configured to receive first artery parameter data and second artery parameter data. The first artery parameter data relates to artery stiffness of a renal artery part of a subject, and the second artery parameter data relates to artery stiffness of a non-renal artery part of the subject. The processing unit is configured to calculate a respective vessel stiffness for the renal artery part based on the first artery parameter data, and to calculate a respective vessel stiffness for the non-renal artery part based on the second artery parameter data, and to determine a response parameter for renal denervation based on the vessel stiffness for the renal artery part and the vessel stiffness for the non-renal artery part. The output unit is configured to provide the response parameter.

According to an example, the first artery parameter data and the second artery parameter data are based on pulse wave velocity.

According to an example, the first artery parameter data and the second artery parameter data are based on at least one of the group of: compliance, distensibility and relative increase in cross-sectional area.

According to an example, the stiffness of the renal artery part is subject to sympathetic overdrive, and the stiffness of the non-renal artery part is unaffected (or at least significantly less affected) by sympathetic overdrive.

According to the present invention, also a system for renal denervation assessment is provided. The system comprises a measurement arrangement and a device for renal denervation preparation according to one of the examples above. The measurement arrangement is configured to detect the first artery parameter data for a renal artery part, and to detect the second artery parameter data for a non-renal artery part, and to provide the first and second artery parameter data to the input unit of the device for renal denervation assessment. As an option, a display is provided to indicate the determined response parameter.

According to an example, the measurement arrangement provides a stiffness parameter at a first location and a stiffness parameter at a second location. In the first location, the measurement arrangement is configured to be arranged at least partly in a renal artery part. In the second location, the measurement arrangement is configured to be arranged in a non-renal artery part.

As an option, a pulse wave velocity is provided as the stiffness parameter for the respective first and second locations.

According to an example, the measurement arrangement comprises at least one sensor of the group of a pressure sensor, a flow velocity sensor and an imaging sensor.

According to an example, the measurement arrangement comprises at least a first sensor unit and a second sensor unit.

According to an aspect, it is provided to assess the likeliness that a patient with resistant hypertension will respond to renal denervation, based on arterial stiffness measurements in both the renal artery and another artery (e.g. the aorta) being a non-renal artery. This enables distinguishing between the effect of stiffening of the artery due to neural stimulation and systemic arteriosclerosis. In an example, a catheter or guide-wire with two sensing units is provided, one located in the aorta the other in the renal artery (or a single sensing unit on a guide-wire/catheter that is moved between the two locations). Further, an acquisition "box" and a respective processing algorithm is provided that collects the signals of the sensing units, calculates the vessel stiffness at the two locations, determines a single parameter based on the two calculated stiffness values and classifies the patient as responder or non-responder of renal denervation purely based on that value. This provided indicator or index or value can then be used for diagnostic steps performed by a surgeon or other qualified person. In a further example, a visualization unit is provided that shows the determined parameter on a screen, and/or indicates the likeliness that the patient will respond to renal denervation with providing e.g. a value or figure.

In an example, a medical interventional device (catheter or guidewire) is provided with at least two sensing units. The units are spaced apart such that one unit can be placed inside the renal artery while the other is placed in another artery, e.g. in the aorta with a distance of e.g. 10 cm. Each sensing unit is capable of assessing the stiffness of the respective vessel. As an example, the stiffness can be assessed with two pressure sensors. A signal acquisition box collects the signals of the sensing elements. The collected signals are processed such that a measure of the stiffness is determined for both arteries.

Based on the combination of the stiffness in both arteries, a parameter is determined that enables stratifying patients with hypertension for renal denervation, i.e. whether a patient is likely to respond to renal denervation. Based on this parameter, a visualization unit indicates the likeliness that the patient will respond to renal denervation.

These and other aspects of the present invention will become apparent from and be elucidated with reference to the embodiments described hereinafter.

BRIEF DESCRIPTION OF THE DRAWINGS

Exemplary embodiments of the invention will be described in the following with reference to the following drawings:

> Fig. 1 schematically shows an example of a device for renal denervation preparation.
> Fig. 2 schematically shows an example of a system for renal denervation assessment.
> Fig. 3 schematically shows a part of a further example of the system for renal denervation assessment in the context of a vascular system.
> Fig. 4 shows a further example in the context of a vascular system.
> Fig. 5 shows a graph in relation with pulse wave velocity measurements of the arrangement shown in Fig. 4.
> Fig. 6 shows a graph in relation with pulse wave velocity measurement results.
> Fig. 7 shows basic steps of an example of a method for renal denervation preparation.

DETAILED DESCRIPTION OF EMBODIMENTS

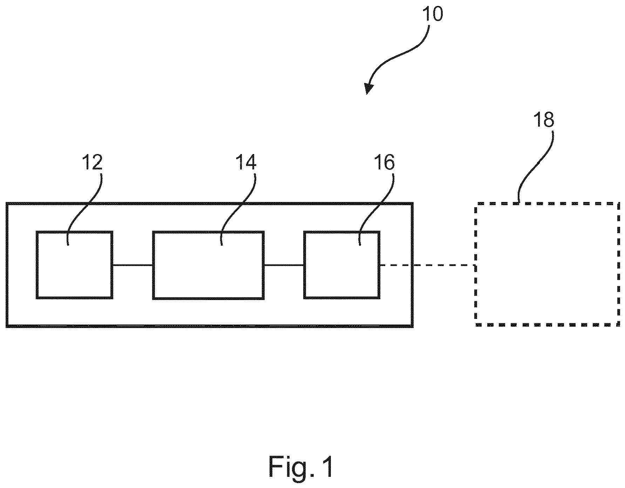

Fig. 1 shows a device or apparatus 10 for renal denervation preparation. The apparatus 10 comprises an input unit 12, a processing unit 14 and an output unit 16. The input unit 12 is configured to receive first artery parameter data and second artery parameter data. The first artery parameter data relates to artery stiffness of a renal artery part of a subject. Further, the second artery parameter data relates to artery stiffness of a non-renal artery part of the subject. The processing unit 14 is configured to calculate a respective vessel stiffness for the renal artery part based on the first artery parameter data. The processing unit 14 is also configured to calculate a respective vessel stiffness for the non-renal artery part based on the second artery parameter data. The processing unit 14 is further configured to determine a response parameter for renal denervation based on the vessel stiffness for the renal artery part and the vessel stiffness for the non-renal artery part. The output unit 16 is configured to provide the response parameter.

The "device for renal denervation preparation" can also be referred to as "inspection device for renal denervation assessment". Also, the terms "device for renal denervation outcome prediction", "device for pre-checking renal denervation" or "analysis device for renal denervation" can be used. In an example, the device for renal denervation preparation also relates to stratification before renal denaturation. The device for renal denervation preparation refers to inspecting, preparing, examining, investigating, analyzing, testing or checking before and after renal denervation, or even during renal denervation.

In an example, taking data from two different locations supports in better distinguishing between hypertension resulting from system arteriosclerosis and hypertension resulting from sympathetic overdrive. Two measurements are taken at the same time or at near same time. The two measurements are then combined into the determined index.

For measuring stiffness, different data can be used. In an example, the pulse wave velocity is used.

The output unit 16 may be connected to a display 18, indicated with hashed lines as an option in Fig. 1, to present the determined response parameter.

It is noted that the terms "first" and "second" are used to distinguish between the two e.g. parameters, locations, units. However, this does not necessarily mean a restriction to a respective order.

The term "subject" may also be referred to as individual. The "subject" may further also be referred to as patient, although it is noted that this term does not indicate whether any illness or disease is actually present with the subject.

In another example, not shown in detail, the first artery parameter data and the second artery parameter data are based on pulse wave velocity.

In an example, the first artery parameter data and the second artery parameter data relate to the stiffness of the respective artery part and the stiffness is assessed via pulse wave velocity.

In another example, the first artery parameter data and the second artery parameter data are based on at least one of the group of compliance, distensibility and relative increase in cross-sectional area.

In another example, the first artery parameter data and the second artery parameter data also relate to the stiffness of the respective artery part, but the stiffness is assessed via other measurements and stiffness detecting principles, such as compliance measurement. For example, for compliance measurement, pressure detection and imaging (such as intravascular ultrasound, IVUS) is provided, e.g. by determining $C = dV/dP$, here $V$ = volume and $P$ = pressure. Another example is via distensibility = $dV/VdP$ or via the Young's modulus or also the determination of a relative increase of the vessel cross-sectional area $dA/A$ (=$dV/V$) or radius $dr/r$.

In an option, the stiffness of the renal artery part is subject to sympathetic overdrive, and the stiffness of the non-renal artery part is unaffected by sympathetic overdrive.

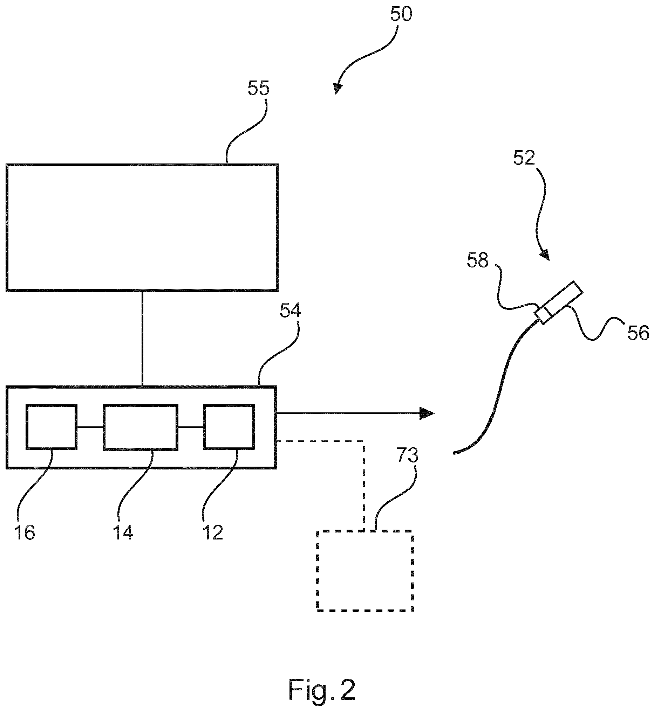

Fig. 2 shows a system 50 for renal denervation assessment. The system 50 comprises a measurement arrangement 52. The system 50 also comprises an example 54 of the device or apparatus 10 for renal denervation preparation according to one of the examples above. The measurement arrangement 52 is configured to detect the first artery parameter data for a renal artery part. The measurement arrangement 52 is also configured to detect the second artery parameter data for a non-renal artery part. The measurement arrangement 52 is still further configured to provide the first and second artery parameter data to the input unit 12 of the device for renal denervation assessment. As an option, a display 55 is provided to indicate the determined response parameter.

The measurement arrangement 52 measures the first artery parameter data at the first location in a vessel structure of the object and the second parameter data at the second location in the vessel structure of the object. The first data is measured in the renal artery part and the second data is measured in the non-renal artery part.

In an example, tracking of the measurement arrangement 52 is provided during measurement.

The measurement arrangement 52 is thus capable of detecting the respective first and second artery parameter data and to provide the parameter as data to the device for renal denervation assessment processing unit, i.e. via the input unit 12 to the processing unit 14.

In an example, the measurement arrangement 52 comprises at least a first sensor unit and a second sensor unit. The first sensor unit provides the first artery parameter data and the second sensor unit provides the second artery parameter data.

In another example, the measurement arrangement 52 comprises a sensor unit. The sensor unit provides the first artery parameter data and the second artery parameter data. The sensor unit can thus be moved from a first location to a second location. The first location is the renal artery part and the second location is the non-renal artery part.

The system for renal denervation assessment can also be referred to as system for renal denervation preparation or the other terms suggested for the respective device above.

In an example, not further shown, the measurement arrangement 52 provides a stiffness parameter at a first location and a stiffness parameter at a second location. In the first location, the measurement arrangement 52 is configured to be arranged at least partly in a renal artery part, and, in the second location, the measurement arrangement 52 is configured to be arranged in a non-renal artery part.

In an option, a pulse wave velocity is provided as the stiffness parameter for the respective first and second locations.

In an example, when the measurement arrangement 52 comprises the first and the second sensor units, the first sensor unit provides an indicator for the pulse wave velocity at the first location and the second sensor unit provides an indicator for the pulse wave velocity at the second location.

In another example, the first sensor unit provides a stiffness parameter at a first location and the second sensor unit provides a stiffness parameter at a second location. The first sensor unit is configured to be arranged in a renal artery part and the second sensor unit is configured to be arranged in a non-renal artery part. A pulse wave velocity is provided as the stiffness parameter for the respective first and second location. In another option, the first artery parameter data and the second artery parameter data are based on at least one of the group of compliance, distensibility and relative increase in cross-sectional area.

In a further example, also shown as an option, the measurement arrangement 52 comprises at least one sensor unit 56 provided on at least one of the group of a guidewire, a catheter and an intervention tool. Alternatively, or in addition, the measurement arrangement 52 comprises at least one sensor 58 of the group of a pressure sensor, a flow velocity sensor and an imaging sensor. A horizontal arrow indicates the data communication between the example 54 of the apparatus 10 for renal denervation preparation and the measurement arrangement 52.

In an example (not shown in detail), the one or two sensor units are provided on an ablation tool. In another example (also not shown in detail), the interventional device is a renal denervation device.

In still another example (not shown in detail), it is provided that two or more interventional devices are used instead of one. Each device has a sensing unit, i.e. sensor unit, to assess the stiffness. One device is placed in the renal artery and the other in another artery, i.e. a non-renal artery like the aorta.

In still another example, an interventional device, i.e. a guidewire, a catheter, etc., is provided with one sensing unit that can measure stiffness and that is connected to a system that can register the position of the interventional device within the vascular tree (e.g. X-ray, ultrasound, EM tracking, optical shape sensing, in situ camera). The stiffness measurement is performed at the first location of choice, e.g. renal artery or aorta; then the interventional device is brought, either manually or automatically by a motorized pullback, to a second location of choice, e.g. aorta or renal artery. In case of image-based registration, the physician can choose the desired measurement locations in the image. The system automatically recognizes whenever stiffness measurements are performed in both vessels of interest and subsequently calculates the renal denervation effectiveness parameter.

In an example, two measurements are provided for acquiring, i.e. providing, data for two different locations. The two measurements are taken to calculate or determine an indicator or factor for an outcome and effectiveness of renal denervation.

The measurement arrangement 52 provides a data collection arrangement to collect e.g. pulse wave data at the two locations. The sensor units can thus each be referred to as a data collection device.

In an example, the two sensors are provided on a guidewire. In another example, the two sensors are provided on two separate devices. In an example, one device is provided that is moved. For the measurement of the data at the first location and the measurement of the data at the second location, the location of the device is tracked.

In an example, the measurement arrangement 52 comprises at least one sensor unit with at least one sensor of the group mentioned above.

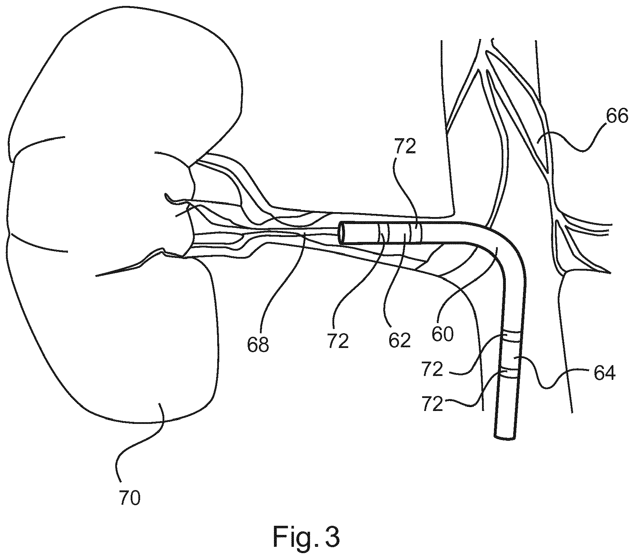

In another example, shown in Fig. 3, the measurement arrangement 52 comprises two sensor units, i.e. a first sensor unit 62 and a second sensor unit 64, each with at least one sensor of the group mentioned above. For example, the first sensor unit 62 provides the first artery parameter data and the second sensor unit 64 provides the second artery parameter data.

In Fig. 3, the first sensor unit 62 and the second sensor unit 64 are provided on a distal end of a flexible elongate member, such as an interventional device 60, e.g. a catheter or guidewire. The interventional device 60 is inserted into a portion of an aorta 66 being a non-renal artery of a vascular system and is reaching into a renal artery 68, connecting a kidney 70 to the vascular system. The first sensor unit 62 is thus placed in the renal artery 68 to provide the first artery parameter data, and the second sensor unit 64 is thus placed in the non-renal artery 66 to provide the second artery parameter data.

In an example, shown as an option in Fig. 3, four sensors 72 are provided and the first sensing unit 62 and the second sensing unit 64 are each provided with two separate sensors of the four sensors 72.

In an example, at least one of the first or second sensing units is provided with two pressure sensors arranged in a known distance. For example, the two sensors are arranged on a common support. The pressure sensors can detect pressure waves by a change in the pressure, and, under consideration of the distance, the pulse wave velocity can be determined.

In another example, at least one of the first or second sensing units is provided with two flow velocity sensors arranged in a known distance. For example, the two sensors are arranged on a common support. Since a pulse wave is resulting not only in a change of the pressure, but also a change in the flow, i.e. the flow velocity, the change in the flow can also form a basis to detect a passing wave. The flow velocity sensors can thus detect pressure waves by a change in the flow velocity, and under consideration of the distance, the pulse wave velocity can be determined.

In a further example, at least one of the first or second sensing units is provided with two imaging sensors arranged in a known distance. For example, the two sensors are arranged on a common support. Since a pulse wave is resulting in a temporal geometric change of the vessel though which the wave passes, e.g. a change in the vessel's diameter, the change in the geometry can also form a basis to detect a passing wave. The imaging sensors can thus detect pressure waves by a change in the geometry, e.g. an increasing vessel diameter, and under consideration of the distance, the pulse wave velocity can be determined.

The imaging sensors may be provided as ultrasound imaging sensors that can be arranged inside a vessel structure, e.g. IVUS and/or OCT.

The imaging sensors may also be provided as optical cameras that can be arranged inside a vessel structure.

In a still further example, at least one of the first or second sensing units is provided with one pressure sensor and one flow velocity sensor arranged in a known distance. However, it is noted that it is also possible to determine PWV with pressure and flow sensors at the same location or even at an unknown distance.

$$PWV = \frac{1}{\rho}\frac{dP}{dU}$$

or

$$PWV = \frac{1}{\rho}\sqrt{\frac{\sum dP^2}{\sum dU^2}}$$

Then these relations can be used to determine PWV; the first at the same location and the second at the same or unknown distance.

In a further example, at least one of the first or second sensing units is provided with one pressure sensor and one imaging sensor arranged in a known distance. Here it is also possible to determine PWV with pressure and cross-sectional (imaging) sensors at the same location or very close to each other.

$$PWV = \sqrt{\frac{1}{\rho D}} \quad ,$$

with

$$D = \frac{dV}{VdP}$$

In a still further example, at least one of the first or second sensing units is provided with one flow velocity sensor and one imaging sensor arranged in a known distance.

In all combinations, according to these examples, the sensors are configured to detect an arrival of a pulse wave. In some examples, considering the distance of the two measurement points, the pulse wave velocity can be determined. However, in other examples, the pulse wave velocity can also be determined with pressure and flow sensors at the same location or even at an unknown distance.

In another example, the first sensing unit and the second sensing unit are each provided with two sensors, wherein one sensor is provided as a shared sensor such that the measurement arrangement 52 comprises three sensors.

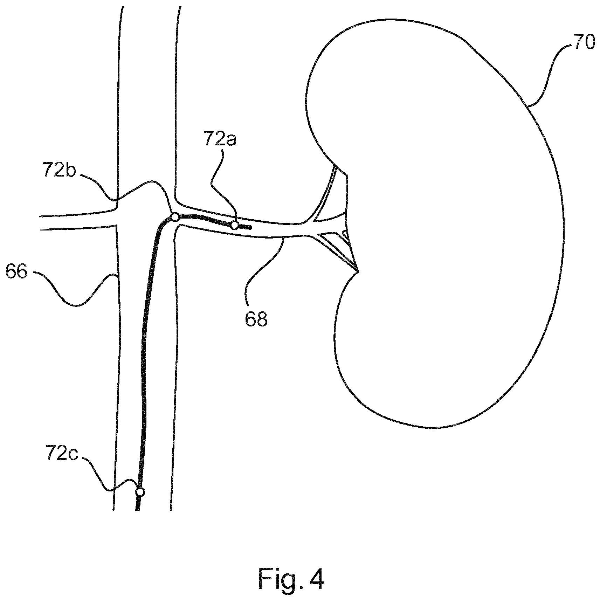

For example, shown in Fig. 4, at least three sensors are provided for the measurement arrangement. In an option, a first sensor 72a and a second sensor 72b provide the first artery parameter data, and the second sensor and a third sensor 72c provide the second artery parameter data. The second sensor 72b is provided as a shared sensor. In an example, the at least three sensors are provided for the first sensing unit and the second sensing unit. The concept of the shared sensor results in that the sensing units are overlapping.

For example, the three sensors are provided as three pressure sensors, i.e. as first, second and third pressure sensor. The first of the three pressure sensors is assigned to the first sensing unit. The second of the three pressure sensors is assigned to both the first sensing unit and the second sensing unit. The third of the three pressure sensors is assigned to the second sensing unit.

In a further example, an intravascular device is provided with the three pressure sensors 72a, 72b, 72c spaced apart at known distances. The device is to be placed (as in Fig. 4) so that the central pressure sensor is located at the branching of the renal artery from the aorta, the distal pressure sensor is located in the renal artery and the proximal pressure sensor is located in the aorta or the femoral artery.

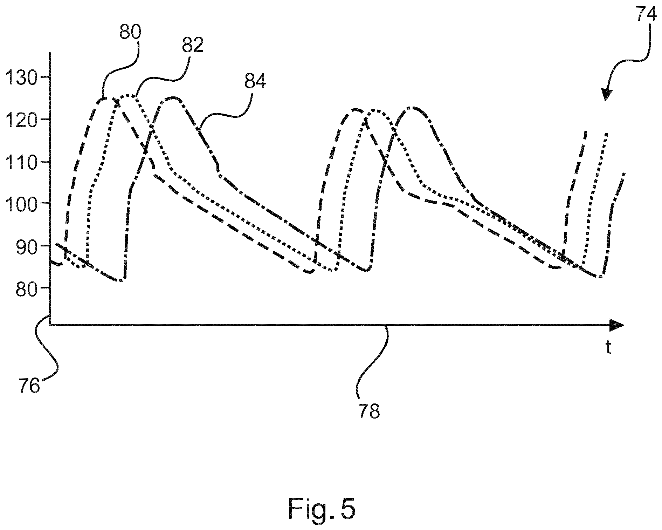

Fig. 5 shows a first graph 74 in relation with the pulse wave velocity measurements of the arrangement with the three pressure sensors 72a, 72b, 72c shown also in Fig. 4. A vertical axis 76 indicates the arterial pressure, and a horizontal axis 78 indicates the time. A first measurement line 80 relates to the central sensor, i.e. the second sensor 72b; a second measurement line 82 relates to the renal sensor, i.e. the first sensor 72a; and a third measurement line 84 relates to the aorta sensor, i.e. the third sensor 72c. With these curves, it is possible to determine a renal artery delay, e.g. between the first and the second measurement lines, and to determine an aorta delay, e.g. between the first and the third measurement lines. When considering the known distances of the sensors, the two different pulse wave velocities can be determined, based on PWV being the ratio of the spatial distance of the sensors and the difference in time:

$$PWV = \frac{\Delta x}{\Delta t}$$

and these can then be further processed for determining the response parameter.

The device can measure PWV in the renal artery and in the lower aorta by timing pressure pulses coming from the heart. Because the central pressure sensor is closest to the heart, it will measure each pressure pulse first. The pressure pulses measured by the distal pressure sensor and the proximal pressure sensor will be delayed, with the delay depending on the distance between the sensors from the central sensor and the pulse wave velocities in the renal artery and the lower aorta respectively. The PWV in the renal artery can be estimated as: $PWV_{RA}$ = (Pulse delay between central and distal sensor)/(Distance between central and distal sensor), while the pulse wave velocity in the lower aorta can be estimated as: $PWV_{Aorta}$ = (Pulse delay between central and proximal sensor)/(Distance between central and proximal sensor). The measured pulse wave velocities will be used as described above.

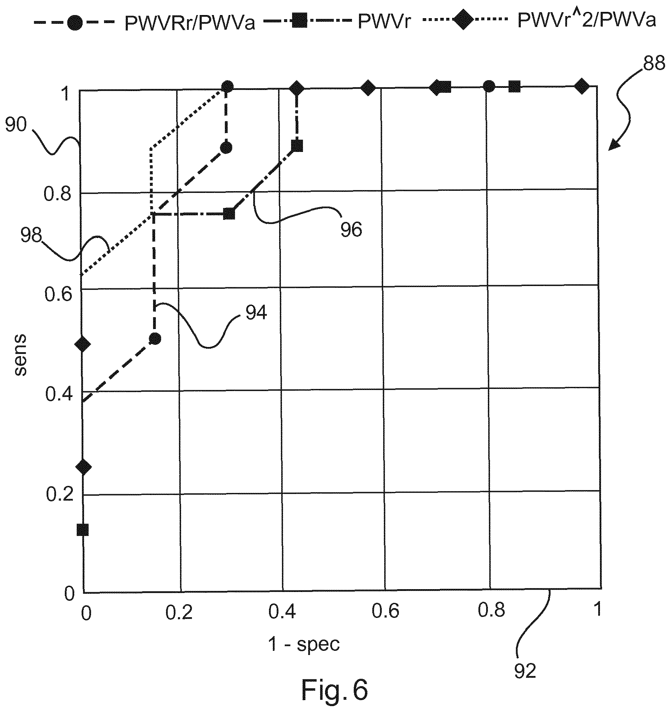

In a still further example, the patient stratification parameter is a ratio of the PWV in the renal artery and another artery, e.g. PWVr/PWVa or PWVr^2/PWVa (or the inverse). Here, PWVr is the PWV in the renal artery and PWVa is the PWV in another artery (aorta). In an example, a Receiver Operating Characteristic (ROC) curve is shown in Fig. 6 for the ability to correctly classify patient based on the parameters, compared to a single PWV parameter in the renal artery.

Fig. 6 shows a second graph 88 with "sensitivity" values on a vertical axis 90, and "1-specificity" values on a horizontal axis 92. A first curve 94 indicates a ratio of a pulse wave velocity in a renal artery (PWVr) and a pulse wave velocity in a non-renal (or other) artery (PWVa), i.e. the first curve relates to PWVr/PWVa. A second curve 96 shows PWVr, i.e. the pulse wave velocity in a renal artery. A third curve 98 indicates PWVr^2/PWVa. All curves are aligned and overlap in the upper right portion of the graph 88.

Shown as an option in Fig. 2 with hashed lines, a respiratory phase detection device 73 is provided. The first artery parameter data and the second artery parameter data are aligned to the detected respiratory phase. The alignment is done based on signals provided by the respiratory phase detection device. In one example, an activation of the first sensor unit to provide the first artery parameter data and an activation of the second sensor unit to provide the second artery parameter data are aligned to the detected respiratory phase. The alignment is thus provided during processing, i.e. during the active measurement.

In another example, the alignment is provided in post-processing, e.g. first and second artery parameter data are collected over one or more breathing cycles and the respectively tagged data is aligned accordingly.

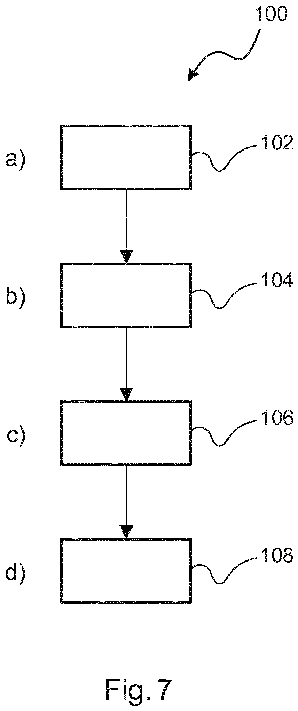

Fig. 7 shows a method 100 for renal denervation preparation. The method 100 comprises the following steps: In a first step 102, also referred to as step a), first artery parameter data and second artery parameter data are received. The first artery parameter data relates to artery stiffness of a renal artery part, and the second artery parameter data relates to artery stiffness of a non-renal artery part. In a second step 104, also referred to as step b), a respective vessel stiffness for the renal artery part is calculated based on the first artery parameter data; and a respective vessel stiffness for the non-renal artery part based on the second artery parameter data. In a third step 106, also referred to as step c), a response parameter for renal denervation is determined based on the vessel stiffness for the renal artery part, and the vessel stiffness for the non-renal artery part is determined. In a fourth step 108, also referred to as step d), the response parameter is provided.

In an example, in step a), the first artery parameter data relates to artery stiffness of a renal artery part, the stiffness of which artery part is subject to sympathetic overdrive; and the second artery parameter data relates to artery stiffness of a non-renal artery part, the stiffness of which artery part is unaffected by sympathetic overdrive.

The measurement at two different locations provides invasive renal artery stiffness (or distensibility) measurements. This results in an improved knowledge and assessment of the effect of e.g. the sympathetic overdrive on the arterial tone. This information can be used to predict renal denervation efficacy at a pre- or also post-treatment stage. As an example, the arterial stiffness is determined using pulse wave velocity measurements. Pulse wave velocity is the transmission speed of pressure/flow waves, e.g. generated by the beating heart, through the arteries. In an example, the pulse wave velocity is determined by the ability of the vessel to expand, i.e. distensibility D, according to the following relation:

$$PWV = \sqrt{\frac{1}{\rho D}} \quad ,$$

with

$$D = \frac{dV}{V dP}$$

with V the vascular volume, P the pressure, and $\rho$ the blood density. From this relation, the so-called Moens-Korteweg equation can be derived:

$$PWV = \sqrt{\frac{E \cdot h}{d \cdot \rho}}$$

here, E is the Young's modulus, *d* is the vessel diameter, and *h* is the wall thickness.

In another example the PWV is determined by the ratio of pressure to flow changes, according either of the following relations:

$$PWV = \frac{1}{\rho}\frac{dP}{dU}$$

or

$$PWV = \frac{1}{\rho}\sqrt{\frac{\sum dP^2}{\sum dU^2}}$$

here, *dP* is the change in pressure per time unit and *dU* is the change in flow per time unit.

By assessing the pulse wave velocity, the stiffness of arteries can therefore be quantified. A typical value of the pulse wave velocity in the renal artery for classifying a patient for being eligible for and responding to renal artery denervation treatment is about 10 m/s. Renal denervation in patents with a too low pulse wave velocity in the renal artery (e.g. lower than the typical value by max. 20%) does not lead to a longterm hypertension reduction.

The vessel parts being subject to sympathetic overdrive can also be referred to as a vessel parts being affected by sympathetic overdrive. The renal artery part is subject to sympathetic overdrive with a higher amount or degree than the non-renal artery part.

The artery parameter can also be provided as distensibility parameter in form of an artery distensibility value. The artery parameter can be referred to as stiffness indicator. The arteries that are subject to sympathetic overdrive can also be referred to as arteries being subject to neural stimulation.

In an example, the response parameter is used for further classification in view of likeliness that a subject will respond to renal denervation.

The response parameter for renal denervation is also referred to as a response index for renal denervation. The physiological quantity is known as pulse wave velocity. In an example, two measurements for stiffness are used for providing an indicator or factor for the likeliness that renal denervation may have an effect in the attempt to reduce hypertension.

The arrangement of two sensors on a guidewire can thus be referred to as "measure of stiffness".

The stiffness is related to the elastic modulus.

In an example, the stiffness measurements are performed in conjunction with a measurement of the respiratory phase, allowing gating of the stiffness measurements in both measurement locations, such that the renal denervation effectiveness parameter can be calculated based on the same respiratory phase. Alternatively, breath holding techniques may be used to ensure that the two stiffness measurements are well comparable.

Alternatively, the stiffness measurements are prolonged at each location, so as to compute an average stiffness over a longer time, so that the measurement is less sensitive to e.g. respiratory variations.

In another example, the effect of the renal denervation treatment on the renal denervation effectiveness parameter is monitored during and after the treatment to guide treatments decision and assess the effectiveness.

According to an aspect, in the non-renal artery part, the stiffness of the artery part results mainly from systemic arteriosclerosis.

As an option, the first artery parameter data and the second artery parameter data are based on pulse wave velocity. Alternatively, or in addition, the first artery parameter data and the second artery parameter data are based on measurements from the same point in time.

In an example, the artery parameter is thus assessed via the pulse wave velocity.

The term "same" point in time relates to the exact same point in time and a deviation of +/- 0.5 s (seconds).

In an example, the first artery parameter data and the second artery parameter data are measured ion the same heart cycle.

In an example, a predetermined delay or offset of maximum 0.5 s, e.g. 0.1 s, for example of maximum 0.01 s is provided for the two measurements at the two locations.

In an example, not further shown, for step a), the first artery parameter data is measured at a first location in a vessel structure of an object and the second parameter data is measured at a second location in a vessel structure of an object. The first data is measured in a renal artery part and the second data is measured in a non-renal artery part.

In an example, the non-renal artery part is an aorta part.

In an example, the first data is measured upstream (in relation to the blood flow) and the second data is measured downstream (in relation to the blood flow).

In another example, the first data is measured downstream (in relation to the blood flow) and the second data is measured upstream (in relation to the blood flow).

In an example, the first artery parameter data and the second artery parameter data are provided by two different devices.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items recited in the claims. The mere fact that certain measures are re-cited in mutually different dependent claims does not indicate that a combination of these measures cannot be used to advantage. Any reference signs in the claims should not be construed as limiting the scope.

**Claims**

1. An apparatus (10) for renal denervation preparation, comprising:

   - an input unit (12);
   - a processing unit (14); and
   - an output unit (16);

   wherein the input unit is configured to receive first artery parameter data and second artery parameter data; wherein the first artery parameter data relates to artery stiffness of a renal artery part of a subject; and the second artery parameter data relates to artery stiffness of a non-renal artery part of the subject; wherein the processing unit is configured to calculate a respective vessel stiffness for the renal artery part based on the first artery parameter data; and to calculate a respective vessel stiffness for the non-renal artery part based on the second artery parameter data; and to determine a response parameter for renal denervation based on the vessel stiffness for the renal artery part and the vessel stiffness for the non-renal artery part; and
   wherein the output unit is configured to provide the response parameter.

2. Apparatus according to claim 1, wherein the first artery parameter data and the second artery parameter data are based on pulse wave velocity.

3. Apparatus according to claim 1 or 2, wherein the first artery parameter data and the second artery parameter data are based on at least one of the group of: compliance, distensibility and relative increase in cross-sectional area.

4. Apparatus according to claim 1, 2 or 3, wherein the stiffness of the renal artery part is subject to sympathetic overdrive, and the stiffness of the non-renal artery part is unaffected by sympathetic overdrive.

5. A system (50) for renal denervation assessment, comprising:

- a measurement arrangement (52); and
- an apparatus (54) for renal denervation preparation according to one of the claims above;

wherein the measurement arrangement is configured to detect the first artery parameter data for a renal artery part; and to detect the second artery parameter data for a non-renal artery part; and to provide the first and second artery parameter data to the input unit of the apparatus for renal denervation assessment; and
wherein, preferably, a display (55) is provided to indicate the determined response parameter.

**6.** System according to claim 5, wherein the measurement arrangement provides a stiffness parameter at a first location and a stiffness parameter at a second location; and
wherein, in the first location, the measurement arrangement is configured to be arranged at least partly in a renal artery part and, in the second location, the measurement arrangement is configured to be arranged in a non-renal artery part; and
wherein, preferably, a pulse wave velocity is provided as the stiffness parameter for the respective first and second locations.

**7.** System according to claim 5 or 6, wherein the measurement arrangement comprises:

i) at least one sensor unit (56) provided on at least one of the group of a guidewire, a catheter and an intervention tool; and/or
ii) at least one sensor (58; 72) of the group of a pressure sensor, a flow velocity sensor and an imaging sensor.

**8.** System according to one of the claims 5 to 7, wherein the measurement arrangement comprises at least a first sensor unit (62) and a second sensor unit (64); and
wherein, preferably, the first sensor unit provides the first artery parameter data and the second sensor unit provides the second artery parameter data.

**9.** System according to one of the claims 5 to 8, wherein at least three sensors are provided for the measurement arrangement; and
wherein, preferably, a first sensor (72a) and a second sensor (72b) provide the first artery parameter data, and the second sensor and a third sensor (72c) provide the second artery parameter data, wherein the second sensor is provided as a shared sensor.

**10.** System according to one of the claims 5 to 9, wherein a respiratory phase detection device (73) is provided;

and
wherein the first artery parameter data and the second artery parameter data are aligned to the detected respiratory phase.

**11.** A method (100) for renal denervation preparation, comprising the following steps:

a) receiving (102) first artery parameter data and second artery parameter data;
wherein the first artery parameter data relates to artery stiffness of a renal artery part; and
wherein the second artery parameter data relates to artery stiffness of a non-renal artery part;
b) calculating (104) a respective vessel stiffness for the renal artery part based on the first artery parameter data; and calculating a respective vessel stiffness for the non-renal artery part based on the second artery parameter data;
c) determining (106) a response parameter for renal denervation based on the vessel stiffness for the renal artery part and the vessel stiffness for the non-renal artery part; and
d) providing (108) the response parameter.

**12.** Method according to claim 11, wherein the first artery parameter data and the second artery parameter data are based on pulse wave velocity; and/or
wherein the first artery parameter data and the second artery parameter data are based on measurements from the same point in time.

**13.** Method according claim 11 or 12, wherein for step a), the first artery parameter data is measured at a first location in a vessel structure of an object and the second parameter data is measured at a second location in a vessel structure of an object; and
wherein the first data is measured in a renal artery part and the second data is measured in a non-renal artery part.

**14.** A computer program element for controlling an apparatus according to one of the claims 1 to 10, which, when being executed by a processing unit, is adapted to perform the method steps of one of the claims 11 to 13.

**15.** A computer readable medium having stored the program element of claim 14.

Fig. 1

Fig. 2

Fig. 3

Fig. 4

Fig. 5

Fig.6

100

a) ⌐‾‾‾‾‾‾‾¬ ⌐ 102

b) ⌐‾‾‾‾‾‾‾¬ ⌐ 104

c) ⌐‾‾‾‾‾‾‾¬ ⌐ 106

d) ⌐‾‾‾‾‾‾‾¬ ⌐ 108

Fig. 7

Europäisches
Patentamt

European
Patent Office

Office européen
des brevets

# EUROPEAN SEARCH REPORT

Application Number

EP 18 18 9998

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/198813 A1 (KONINKLIJKE PHILIPS NV [NL]) 23 November 2017 (2017-11-23)<br>* abstract *<br>* page 4, line 12 - page 5, line 10 *<br>* page 7, line 3 - line 6 *<br>* page 9, line 21 - page 11, line 8 *<br>* page 12, line 3 - line 16 *<br>* page 16, line 6 - page 17, line 29 *<br>* page 21, line 7 - line 18 *<br>* page 23, line 19 - page 24, line 4 *<br>* figures 1-3 * | 1-15 | INV.<br>A61B5/02<br>A61B5/021<br>A61B5/0215 |
| A | US 2014/012133 A1 (SVERDLIK ARIEL [IL] ET AL) 9 January 2014 (2014-01-09)<br>* abstract *<br>* paragraph [0004] - paragraph [0005] *<br>* figures 7,8 * | 1-15 | |
| A | KARL FENGLER ET AL: "Pulse Wave Velocity Predicts Response to Renal Denervation in Isolated Systolic Hypertension",<br>JOURNAL OF THE AMERICAN HEART ASSOCIATION,<br>vol. 6, no. 5, 5 May 2017 (2017-05-05),<br>XP055547892,<br>ISSN: 2047-9980, DOI:<br>10.1161/JAHA.117.005879<br>* abstract *<br>* pages 1,2,6 * | 1-15 | TECHNICAL FIELDS SEARCHED (IPC)<br><br>A61B |

-/--

The present search report has been drawn up for all claims

| Place of search | Date of completion of the search | Examiner |
|---|---|---|
| The Hague | 28 January 2019 | Van der Haegen, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 18 18 9998

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | THOMAS OKON ET AL: "Invasive aortic pulse wave velocity as a marker for arterial stiffness predicts outcome of renal sympathetic denervation", EUROINTERVENTION, vol. 12, no. 5, 1 August 2016 (2016-08-01), pages e684-e692, XP055547428, FR ISSN: 1774-024X, DOI: 10.4244/EIJV12I5A110 * the whole document * | 1-15 | |
| A | FENGLER KARL ET AL: "Cardiac magnetic resonance assessment of central and peripheral vascular function in patients undergoing renal sympathetic denervation as predictor for blood pressure response", CLINICAL RESEARCH IN CARDIOLOGY, SPRINGER BERLIN HEIDELBERG, BERLIN/HEIDELBERG, vol. 107, no. 10, 9 May 2018 (2018-05-09), pages 945-955, XP036600918, ISSN: 1861-0684, DOI: 10.1007/S00392-018-1267-6 [retrieved on 2018-05-09] * abstract * * page 946 - page 948 * * page 950 - page 951 * | 1-15 | TECHNICAL FIELDS SEARCHED (IPC) |
| A | US 2013/325000 A1 (BATES MARK C [US]) 5 December 2013 (2013-12-05) * abstract * * figures 2A-B * | 1-15 | |

The present search report has been drawn up for all claims

| Place of search | Date of completion of the search | Examiner |
|---|---|---|
| The Hague | 28 January 2019 | Van der Haegen, D |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 18 9998

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2019

| Patent document cited in search report | Publication date | Patent family member(s) | Publication date |
|---|---|---|---|
| WO 2017198813 A1 | 23-11-2017 | CN 109152540 A <br> WO 2017198813 A1 | 04-01-2019 <br> 23-11-2017 |
| US 2014012133 A1 | 09-01-2014 | NONE | |
| US 2013325000 A1 | 05-12-2013 | US 2013325000 A1 <br> WO 2013181278 A1 | 05-12-2013 <br> 05-12-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017198490 A1